Europäisches Patentamt

European Patent Office                    (11) Numéro de publication: **0 301 431**

Office européen des brevets                                                    **A1**

(12)                              **DEMANDE DE BREVET EUROPEEN**

(21) Numéro de dépôt: **88111835.0**          (51) Int. Cl.⁴ **A41B 13/02**

(22) Date de dépôt: **22.07.88**

(30) Priorité: **30.07.87 FR 8710835**          (71) Demandeur: **PEAUDOUCE**
                                              **59, Rue de la Vignette**
                                              **F-59126 Linselles(FR)**

(43) Date de publication de la demande:
    **01.02.89 Bulletin 89/05**              (72) Inventeur: **Villez, Yves**
                                              **13, Rue Albert Cames**
                                              **F-59126 Linselles(FR)**

(84) Etats contractants désignés:
    **AT BE CH DE ES FR GB GR IT LI LU NL SE**     (74) Mandataire: **Casalonga, Axel et al**
                                              **BUREAU D.A. CASALONGA - JOSSE**
                                              **Morassistrasse 8**
                                              **D-8000 Munich 5(DE)**

(54) **Couche-culotte à élastiques longitudinaux et procédé de fabrication en continu de telles couches-culottes.**

(57) Couche-culotte caractérisée par le fait que des rubans (40) sont placés sur la face externe de la feuille imperméable extérieure (29) sur toute la longueur de la feuille imperméable et sur une largeur supérieure à la largeur des éléments élastiques (42), les rubans (40) enserrant les éléments élastiques (42) également disposés sur la face externe de la feuille imperméable extérieure (29).

FIG. 2

# COUCHE-CULOTTE A ELASTIQUES LONGITUDINAUX ET PROCEDE DE FABRICATION EN CONTINU DE TELLES COUCHES-CULOTTES.

La présente invention se rapporte à une couche-culotte du type comprenant une feuille extérieure souple, imperméable aux liquides, dont la face interne est munie de lignes longitudinales de colle, un coussin absorbant disposé sur la face interne de la feuille extérieure de manière que ses deux bords longitudinaux opposés soit en retrait par rapport aux deux bords longitudinaux opposés de la feuille extérieure et que ses bords transversaux opposés soient en retrait par rapport aux deux bords transversaux opposés de la feuille extérieure, une feuille intérieure souple, perméable aux liquides, recouvrant la face interne de la feuille extérieure et le coussin absorbant disposé sur cette face, des éléments élastiques longitudinaux fixés par collage à l'état tendu sur la feuille extérieure, le long de la partie médiane des deux bords longitudinaux opposés de cette feuille, la feuille intérieure étant fixée par collage à la feuille extérieure grâce à un encollage de sa face externe dans la zone de contact des feuilles autour du matelas, et des moyens d'attache pour fermer la couche-culotte autour du corps de l'utilisateur.

L'invention se rapporte également à un procédé de fabrication en continu de telles couches-culottes.

Le procédé connu de fabrication en continu de couches-culottes du type défini ci-dessus consiste

- à dérouler une bande continue de feuille imperméable aux liquides,
- à appliquer des lignes longitudinales continues de colle sur la face supérieure de ladite bande,
- à dérouler des éléments élastiques continus, à encoller lesdits éléments élastiques par intervalles de manière à disposer de sections encollées séparées par des sections non encollées, et à les appliquer à l'état tendu sur la face supérieure de la bande, imperméable entre les lignes de colle au voisinage des deux bords longitudinaux opposés de la bande, de manière à faire adhérer les éléments élastiques par sections successives espacées à la bande, imperméable,
- à déposer successivement, sur la face supérieure de la bande imperméable munie des éléments élastiques, des coussins absorbants individuels de largeur inférieure à la largeur de la bande de manière que les coussins successifs soient disposés sur la bande dans les zones où les éléments élastiques adhèrent à cette dernière et soient espacés les uns des autres dans le sens de la longueur de la bande imperméable,
- à dérouler une bande continue de feuille perméable aux liquides ayant la même largeur que la bande imperméable, à encoller ladite bande sur une face, et à l'appliquer par ladite face encollée sur la face supérieure de la bande imperméable déjà munie des éléments élastiques et des coussins absorbants, et
- à découper successivement dans le sens transversal les deux bandes et les éléments élastiques tendus, entre les coussins successifs espacés, c'est-à-dire dans les sections non encollées des éléments élastiques.

Lors de ce découpage transversal qui fournit les couches-culottes individuelles, les éléments élastiques initialement continus et maintenus à l'état tendu sont sectionnés, de sorte que les parties des éléments élastiques disposées sur chaque couche-culotte de part et d'autre des sections encollées, adhérant à la feuille imperméable, se contractent, c'est-à-dire se détendent librement, alors que les sections encollées adhérant à la feuille extérieure se contractent avec ladite feuille pour donner à cette dernière, dans la zone d'entrejambes de la couche-culotte, l'élasticité requise pour assurer une bonne adaptation au corps de l'utilisateur et une bonne étanchéité.

Toutefois, pour permettre une détente libre des parties non encollées des éléments élastiques lors de ce sectionnement, il est nécessaire de faire également en sorte que ces parties n'adhèrent pas à la feuille perméable encollée. C'est la raison pour laquelle on prévoit jusqu'à présent, dans l'encollage de cette feuille perméable, des zones non encollées à l'endroit des sections non encollées des éléments élastiques avant le sectionnement.

Cela donne donc naissance, sur la couche-culotte, dans la zone comprise entre les bords transversaux espacés du coussin absorbant d'une part, et des feuilles perméable et imperméable d'autre part, à des "tunnels" s'étendant entre ces deux feuilles depuis les bords transversaux du coussin jusqu'aux bords transversaux des feuilles, c'est-à-dire faisant communiquer avec l'extérieur l'espace compris entre les deux feuilles et contenant le coussin absorbant. Ces "tunnels" non seulement favorisent l'échappement vers l'extérieur, par effet de drainage, de l'urine absorbée par le coussin, mais permettent également l'échappement vers l'extérieur de la matière constitutive particulaire du coussin. Tel est le cas par exemple pour des coussins absorbant en pulpe de cellulose défibrée, mais en particulier pour des coussins contenant de la matière superabsorbante en grains qui, bien qu'étant généralement incorporée à une autre matière, par exemple à de la pulpe de cellulose défibrée, se détache souvent très facilement du coussin absorbant et peut, en s'échappant par les "tunnels" en question, parvenir à l'extérieur et en raison de sa présentation sous forme de grains très fins et légers à l'état sec, avoir ici des effets gênants, voire nuisibles pour les utilisateurs.

Dans la demande de brevet n° 86 16 844 qui n'a été publiée qu'après la date dont bénéficie la présente demande, on a préconisé l'utilisation de rubans longitudinaux placés sur la face interne de la feuille impermébale de la couche-culotte à l'endroit des éléments élastiques de façon à définir des "tunnels" à l'intérieur desquels ne peut pas pénétrer la matière constitutive du coussin absorbant disposé au-dessus de ces rubans du fait que les tunnels ne sont ouverts qu'aux deux bords transversaux de la couche-culotte.

Il a été également préconisé de réaliser les rubans formant les tunnels en une matière imperméable aux liquides de façon à éviter toute migration de liquide par l'intermédiaire de ces tunnels qui se trouvent ainsi isolés.

La présente invention a pour objet une couche-culotte du type défini ci-dessus sur laquelle le risque d'échappement vers l'extérieur de matière constitutive du coussin absorbant est empêché avec certitude. L'invention a également pour objet une couche-culotte du type défini ci-dessus sur laquelle l'échappement vers l'extérieur, à l'endroit des bords transversaux de la couche-culotte, des liquides absorbés par le coussin de la couche-culotte est empêché ou pour le moins fortement réduit. L'invention a par ailleurs pour objet une couche-culotte sur laquelle le risque d'échappement de liquide sur les bords latéraux (longitudinaux) du coussin absorbant est empêché ou pour le moins fortement réduit.

Enfin, l'invention a pour objet un procédé de fabrication en continu de telles couches-culottes.

La demanderesse a découvert qu'il était possible de résoudre le problème ainsi posé, en plaçant les rubans longitudinaux ainsi que les éléments élastiques qu'ils recouvrent, non plus sur la face interne de la feuille imperméable mais au contraire sur sa face externe, sensiblement dans la même position transversale. De cette manière, on obtient en effet également une isolation parfaite des tunnels à l'intérieur desquels ne peuvent plus pénétrer ni les liquides ni d'éventuelles matières particulaires incluses dans le coussin absorbant.

Selon l'invention, les rubans sont donc placés sur la face externe de la feuille imperméable, sur toute la longueur de ladite feuille imperméable et sur une largeur supérieure à la largeur des éléments élastiques, les rubans enserrant les éléments élastiques également fixés sur la face externe de la feuille imperméable.

Les rubans peuvent être réalisés en non-tissé ou en une matière imperméable étant entendu que dans ce mode de réalisation l'imperméabilité aux liquides se trouvant dans le matelas absorbant provient non plus de la matière du ruban mais de celle qui constitue la feuille extérieure imperméable. Le choix du matériau pour le ruban dépend donc essentiellement de considérations esthétiques ou d'état de surface pour le contact à l'extérieur de la couche-culotte.

Les éléments élastiques utilisés peuvent être constitués chacun par une bandelette en matière élastique. Ils peuvent également être constitués par une pluralité de brins individuels placés parallèlement les uns par rapport aux autres.

Dans un mode de réalisation préféré, les éléments élastiques sont fixés à l'état tendu par collage dans leur portion médiane, leurs extrémités avant et arrière non fixées étant sans tension et rétractées à l'intérieur des tunnels formés par les rubans.

Les éléments élastiques sont de préférence disposés selon un trajet rectiligne. On notera cependant que l'on pourrait parfaitement envisager d'utiliser des élastiques disposés selon un trajet curviligne, par exemple en suivant la bordure de la couche-culotte ou celle du coussin absorbant.

Il est également possible d'envisager l'utilisation d'éléments élastiques encollés sur toute leur longueur mais ne présentant pas les mêmes caractéristiques d'élasticité dans différentes sections. C'est ainsi qu'il est possible d'utiliser des éléments élastiques exerçant une traction dans leur portion médiane de façon à former des fronces dans la zone d'entre-jambes de la couche-culotte tandis que les portions d'extrémité des mêmes élastiques également fixées par collage sur la face externe de la feuille imperméable n'exercent aucune traction sur cette dernière.

Pour réaliser en continu une couche-culotte conforme à l'invention, on procède de préférence de la manière suivante :
- on déroule en continu deux rubans en feuille écartés l'un de l'autre;
- on applique en continu sur la face supérieure des deux rubans, des éléments élastiques en bande ou en brins continus préalablement encollés au moins partiellement et sous une tension appropriée;
- on déroule une bande continue de feuille imperméable aux liquides;
- on applique en continu sur la face inférieure de la bande continue, les deux rubans continus munis des éléments élastiques ainsi encollés;
- puis on applique en continu des lignes longitudinales de colle espacées transversalement sur la face supérieure de la bande continue imperméable;
- on dépose sur la face supérieure de la bande continue, des coussins absorbants à intervalles réguliers;
- on déroule une bande continue de feuille perméable aux liquides ayant sensiblement la même largeur que la bande continue imperméable et on applique ladite bande perméable sur la face supérieure de la bande imperméable de façon que la bande perméable adhère tout autour des coussins absorbants à la face interne supérieure de la bande imperméable;
- et enfin on découpe transversalement l'ensemble, entre les coussins absorbants successifs.

Lors de la découpe, les portions d'extrémité non encollées des éléments élastiques appliqués à l'état tendu peuvent se rétracter dans les tunnels formés par les rubans encollés sur la face inférieure et externe de la feuille imperméable.

L'invention sera mieux comprise à l'étude d'un mode de réalisation particulier décrit à titre nullement limitatif et illustré par les dessins annexés sur lesquels :

la figure 1 est une vue de la face interne d'une couche-culotte conforme à l'invention représentée, à plat les éléments élastiques étant tendus avec deux arrachements partiels montrant la structure interne de la couche-culotte;

la figure 2 est une coupe à plus grande échelle suivant II-II de la figure 1;

la figure 3 montre les différentes étapes principales de la fabrication en continu des couches-culottes suivant les figures 1 et 2.

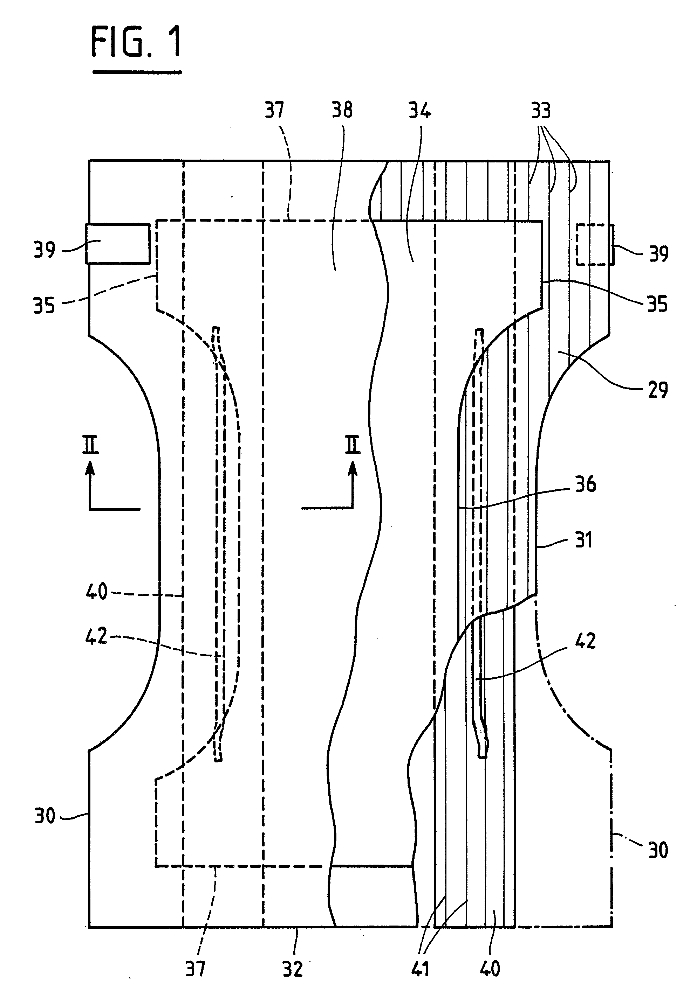

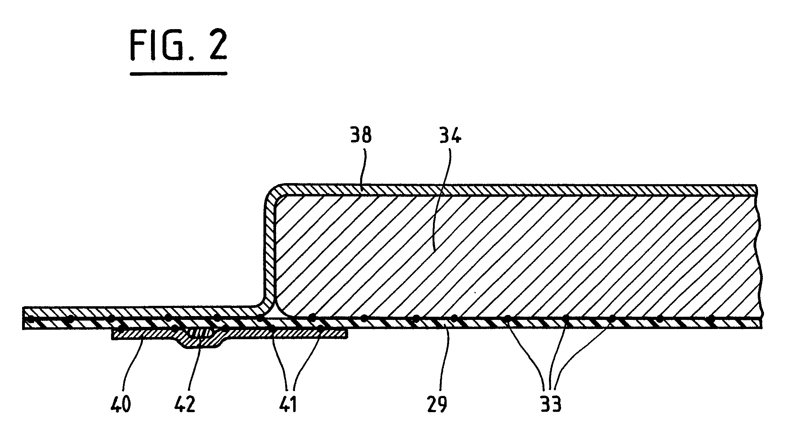

Tel qu'elle est illustrée sur les figures 1 et 2, la couche-culotte comprend une feuille extérieure souple 29 imperméable aux liquides, par exemple une feuille de matière plastique telle que du polyéthylène, présentant une forme générale rectangulaire avec deux bords longitudinaux 30 opposés munis chacun d'une échancrure médiane 31 et deux bords transversaux 32 opposés rectilignes. La feuille 29 est munie sur sa face intérieure, apparente sur la figure 1, d'une multitude de lignes de colle 33 longitudinales espacées.

Un coussin absorbant 34 en forme générale de sablier comporte deux bords longitudinaux 35 opposés munis chacun d'une échancrure médiane 36 et deux bords transversaux 37 opposés et rectilignes et fixés sur la face intérieure de la feuille 29 de manière que ses bords longitudinaux 35 et transversaux 37 se trouvent en retrait par rapport aux bords longitudinaux 30 et transversaux 32 de la feuille 29. Le coussin absorbant 37 peut être constitué par exemple de pulpe de cellulose défibrée, contenant ou non de la matière superabsorbant en grains. La fixation du coussin 37 sur la feuille 29 s'effectue par les lignes de colle 33.

L'ensemble de la feuille 29 est recouvert, sur la face intérieure portant le coussin absorbant 37, d'un voile 38 perméable aux liquides, par exemple un voile de non-tissé. Ce voile 38 peut être muni, sur sa face tournée vers la feuille 29, d'un encollage approprié tel que la feuille 38 adhère au moins à la feuille 29 et sur tout le pourtour du coussin absorbant 34. Cet encollage peut être réalisé sur toute la surface de la feuille 38 auquel cas cette dernière adhère également au coussin absorbant 34 ou être réalisé selon un motif en forme de cadre fermé entourant une fenêtre qui peut par exemple avoir une forme rectangulaire ou la forme du coussin absorbant 34.

Des attaches adhésives 39 de type connu sont fixées sur les deux bords longitudinaux 30 opposés de la couche-culotte dans sa partie arrière pour permettre la fermeture de la couche-culotte autour de la taille de l'utilisateur.

Un ruban 40 réalisé en un matériau souple en feuille par exemple un voile de non-tissé ou un papier enduit ou non, ou encore une feuille de matière plastique, est fixé sur toute la longueur de la feuille 29 sur sa face extérieure comme on peut le voir en particulier sur la figure 2. La fixation est faite par des lignes de colle longitudinales 41. Entre le ruban 40 et la feuille imperméable 29 se trouve un éléments élastique 42 qui est constitué dans l'exemple illustré par une bandelette en matière élastique qui a été fixée à l'état tendu par collage sur la surface interne du ruban longitudinal 40. L'encollage de l'élément élastique 42 est fait uniquement dans la zone médiane qui correspond sensiblement à l'échancrure 36 du matelas absorbant 34 fixé sur la face interne de la feuille imperméable 29. Au contraire les portions d'extrémité de l'élément élastique 42 ne sont pas encollées, Au moment de la découpe transversale qui définit les couches-culottes individuelles, les éléments élastiques 42 se trouvent donc sectionnés au droit des bords transversaux 32 de la feuille imperméable 29. Les extrémités tendues mais non encollées des éléments élastiques 42 se rétractent à l'intérieur du tunnel formé entre le ruban longitudinal 40 et la face externe de la feuille imperméable 29 comme illustré sur la figure 1.

On notera que chaque éléments élastique 42 est placé de façon à se trouver légèrement à l'extérieur du bord de l'échancrure 36 du matelas absorbant 34.

Grâce à la disposition des éléments élastiques 42 et des rubans longitudinaux 40 à l'extérieur de la feuille imperméable 29, il est clair que la matière constitutive du coussin absorbant 34 ne peut en aucune manière pénétrer dans le tunnel défini par les rubans 40 et s'échapper vers l'extérieur par ces tunnels et ce, quelle que soit la matière dont sont constitués les rubans 40.

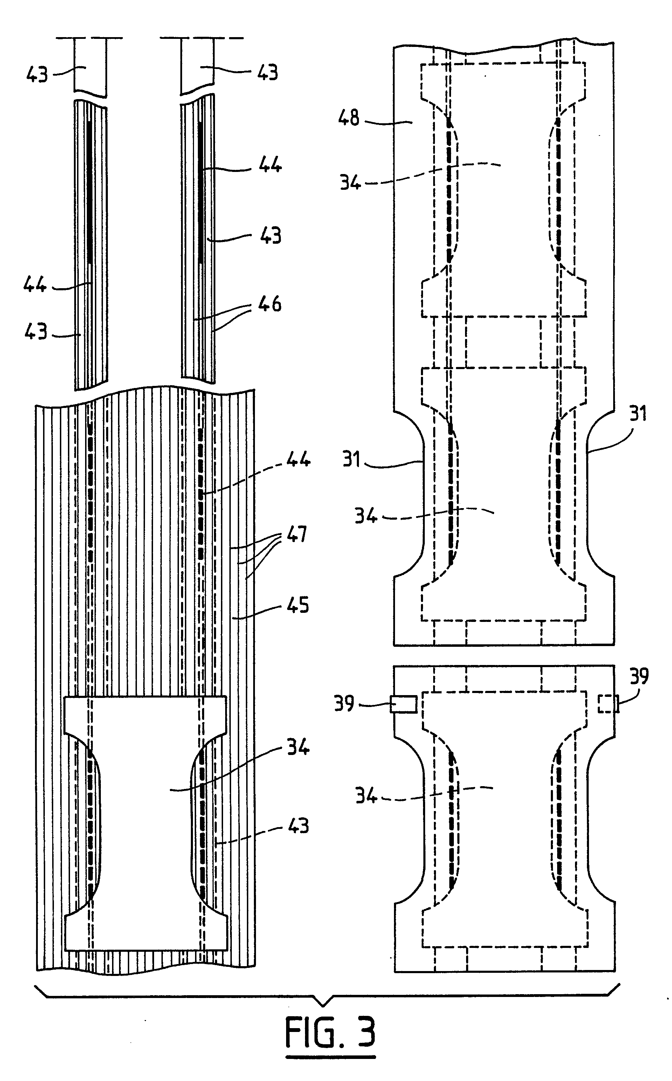

On va maintenant décrire en se référant à la figure 3, un mode de réalisation préféré pour la fabrication en continu des couches-culottes conformes aux figures 1 et 2.

On déroule tout d'abord en continu deux rubans en feuille 43 à un certain écartement l'un de l'autre qui est l'écartement qu'ils devront occuper sur la couche-culotte ultérieurement fabriquée. Sur la face supérieure de ces deux rubans on applique en continu les éléments élastiques en bandelette continue 44 ou en brins continus individuels parallèles entre eux. Ces éléments élastiques continu ont été au préalable encollés, par exemple par sections successives et sont posés avec une tension constante en étant fixés uniquement par sections. Dans d'autres modes de réalisation, on peut envisager un encollage continu avec une variation de la tension de l'élastique continu.

Après cette opération, on déroule en continu une bande 45 de feuille imperméable aux liquides destinée à constituer la feuille imperméable 29 de la couche-culotte et on applique en continu sur la face inférieure de la bande continue 45 les deux rubans continus 43 munis des éléments élastiques 44 encollés comme il a été dit précédemment. La fixation se fait par exemple au moyen de lignes de collage 46 appliquées en continu directement sur les rubans 43 ou au contraire sur la face inférieure de la bande continue 45.

Sur la face supérieure de la bande continue imperméable 45, on applique une multitude de lignes de colle longitudinales 47 puis on dépose les coussins absorbants 34 préalablement découpés afin d'avoir une forme analogue à celle d'un sablier. On déroule ensuite une bande continue 48 de feuille souple perméable aux liquides ayant la même largeur que la bande 45 et on l'applique en l'encollant sur la face supérieure de la bande 45 par-dessus les coussins 34.

On découpe dans les deux bandes 45 et 48, sensiblement dans la zone médiane de chaque coussin 34 deux échancrures latérales opposées 31. Après avoir ensuite fixé les attaches adhésives 39 aux endroits appropriés, on découpe transversalement l'ensemble ainsi formé ce qui provoque la rétraction des zones d'extrémité des éléments élastiques continus 44 dans leurs portions non encollées.

**Revendications**

1. Couche-culotte du type comprenant une feuille extérieure souple, imperméable aux liquides dont la face interne est munie de lignes longitudinales de colle, un coussin absorbant disposé sur la face interne de la feuille extérieure de manière que ses deux bords longitudinaux opposés soient en retrait par rapport aux deux bords longitudinaux opposés de la feuille extérieure et que ses bords transversaux opposés soient en retrait par rapport aux deux bords transversaux opposés de la feuille extérieure, une feuille intérieure souple, perméable aux liquides, recouvrant la face interne de la feuille extérieure et le coussin absorbant disposé sur cette face, des éléments élastiques longitudinaux fixés par collage à l'état tendu sur la feuille extérieure, le long de la partie médiane des deux bords longitudinaux opposés de cette feuille, la feuille intérieure étant fixée par collage à la feuille extérieure grâce à un encollage de sa face externe dans la zone de contact des feuilles autour du coussin, et des moyens d'attache pour fermer la couche-culotte autour du corps de l'utilisateur, caractérisée par le fait qu'elle comprend, en outre, deux rubans de feuille souple (40) placés sur la face externe de la feuille imperméable extérieure sur toute la longueur de la feuille imperméable et sur une largeur supérieure à la largeur des éléments élastiques, les rubans enserrant les éléments élastiques (42) également disposés sur la face externe de la feuille imperméable extérieure.

2. Couche-culotte selon la revendication 2, caractérisée par le fait que les rubans sont fixés par collage sur la face externe de la feuille imperméable.

3. Couche-culotte selon les revendications 1 ou 2, caractérisée par le fait que les éléments élastiques sont fixés par collage d'au moins certains sections de leurs longueurs sur la face interne des rubans.

4. Couche-culotte selon l'une quelconque des revendications précédentes, caractérisée par le fait que les rubans sont réalisés en non-tissé;

5. Couche-culotte selon l'une quelconque des revendications précédentes, caractérisée par le fait que le coussin absorbant contient une matière superabsorbante sous forme particulaire.

6. Couche-culotte selon l'une quelconque des revendications précédentes, caractérisée par le fait que les éléments élastiques sont constitués chacun par une bandelette en matière élastique.

7. Couche-culotte selon l'une quelconque des revendications 1 à 5, caractérisée par le fait que les éléments élastiques sont constitués chacun par une pluralité de brins individuels placés parallèlement les uns aux autres.

8. Couche-culotte selon l'une quelconque des revendications précédentes, caractérisée par le fait que les éléments élastiques sont fixés à l'état tendu par collage dans leur portion médiane, leurs extrémités avant et arrière non fixées étant sans tension et rétractées à l'intérieur des tunnels formés par les rubans.

9. Couche-culotte selon l'une quelconque des revendications précédentes, caractérisée par le fait que les éléments élastiques sont rectilignes.

10. Procédé de fabrication en continu de couche-culotte, caractérisée par le fait qu'il comprend les étapes consistant :
- à dérouler deux rubans continus (43) en feuille écartés l'un de l'autre;
- à appliquer en continu sur la face supérieure des deux rubans des éléments élastiques en bande ou en brins continus, préalablement encollés au moins partiellement et sous une tension appropriée; - à dérouler une bande continue (45) de feuille imperméable aux liquides;

- à appliquer en continu sur la face inférieure de la bande continue les deux rubans continus munis des éléments élastiques ainsi encollés;

- à appliquer en continu des lignes longitudinales de colle (47) espacées transversalement sur la face supérieure de ladite bande continue;

- à déposer sur la face supérieure de la bande continue des coussins absorbants (34);

- à dérouler une bande continue (48) perméable aux liquides ayant sensiblement la même largeur que la bande continue imperméable et à appliquer ladite bande perméable sur la face supérieure de la bande imperméable de façon que la bande perméable adhère tout autour des coussins absorbants à la bande imperméable;

- et à découper transversalement l'ensemble entre les coussins absorbants successifs.

## FIG. 1

# FIG. 2

38  34

40  42  41  29  33

EP 0 301 431 A1

## FIG. 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| P,Y | EP-A-0 270 979  (BOUSSAC SAINT FRERES B.S.F.)<br>* En entier * | 1,2,10 | A 41 B   13/02 |
| D,X | | 3-9 | |
| Y | FR-A-2 388 515  (THE PROCTER & GAMBLE CO.)<br>* Page 6, alinéa 3; pages 7,8; page 10, lignes 35-39; page 12, alinéa 1 * | 1,2,10 | |
| A | DE-A-3 319 043  (VEREINIGTE PAPIERWERKE SCHICKEDANZ & CO.)<br>* Page 9, alinéa 3; page 10, alinéa 1; page 11, alinéa 2; page 12, alinéas 1-4; revendications 1-3; figures 1,5,7 * | 1,2,10 | |
| A | EP-A-0 113 976  (UNI-CHARM CORP.)<br>* Page 5, lignes 7-35; pages 6,7; revendications 1,5-9; figures 1-11 * | 1,2,10 | |
| A | US-A-4 353 762  (F.J. BOUDA)<br>* Colonne 3 - colonne 4, ligne 8; figures 1-4 * | 1,2,10 | DOMAINES TECHNIQUES RECHERCHES (Int. Cl.4)<br><br>A 41 B<br>A 61 F |

Le présent rapport a été établi pour toutes les revendications

| Lieu de la recherche | Date d'achèvement de la recherche | Examinateur |
|---|---|---|
| LA HAYE | 25-10-1988 | GARNIER F.M.A.C. |

EPO FORM 1503 03.82 (P0402)